# FASCICULE DE BREVET EUROPEEN

(11) **EP 2 106 755 B1**
(45) Date de publication et mention de la délivrance du brevet: **07.09.2011**
(21) Numéro de dépôt: 09162771.1
(22) Date de dépôt: 14.11.2002
(51) Int. Cl.: A61B 17/12

(54) **Procédé de fabrication d'un dispositif d'occlusion d'un conduit corporel**
Verfahren zur Herstellung einer Okklusionsvorrichtung
Manufacturing process of an occlusion device

(30) Priorité: 19.11.2001 FR 0114803
(43) Date de publication de la demande: 07.10.2009
(62) Demande divisionnaire de: 02793236.7
(73) Titulaire: SOFRADIM PRODUCTION, 01600 Trévoux (FR)
(72) Inventeur: Therin, Michel, 69004, Lyon (FR); Darnis, Thierry, 01480, Frans (FR); Meneghin, Alfredo, 69009, Lyon (FR); Milleret, René, 34000, Montpellier (FR)
(74) Mandataire: Maureau, Philippe

(56) Documents cités:
- EP-A- 0 800 791
- WO-A-94/09705
- WO-A-94/15534
- US-A- 5 310 407
- US-A- 5 690 666

## Description

La présente invention concerne un procédé de frabrication d'un dispositif d'occlusion d'un conduit corporel, notamment d'une veine variqueuse.

Pour le traitement d'une veine variqueuse, la technique dite de "stripping" est habituellement pratiquée. Cette technique consiste à arracher la veine au moyen d'un câble ou d'une sonde. Elle a toutefois pour inconvénient d'être douloureuse et relativement exigeante quant à sa mise en oeuvre.

Des techniques de traitement endo-veineux ont également été envisagées.

La technique dite "CLOSURE" utilise une sonde émettant des ondes radio, ces ondes provoquant un échauffement de la paroi de la veine, et donc une lésion de celle-ci, de laquelle il résulte l'oblitération de la veine. Cette technique permet un bon contrôle du geste opératoire et de la température appliquée localement, et est utilisable pour des veines allant jusqu'à 12 millimètres de diamètre. Elle présente par contre le risque de brûlure de la peau si le patient est maigre, et nécessite une anesthésie générale ou locale avec une sédation forte compte tenu des douleurs qu'elle implique. De plus, cette technique n'est pas indiquée si la veine présente des zones fortement dilatées, n'est pas utilisable sur les veines collatérales et sur la veine saphène externe, ni sur les récidives, et a un coût de mise en oeuvre relativement élevé.

La technique dite "EVLT" utilise une fibre optique reliée à un laser. Cette technique est plus simple à mettre en oeuvre et moins coûteuse. Elle présente toutefois des risques de brûlure et implique des suites plus douloureuses que la technique précédente, compte tenu de la température de traitement, plus élevée. En outre, cette technique n'est pas utilisable pour certaines veines, telles que les veines anévrismales, la veine saphène externe ou les veines collatérales, et le laser a un coût élevé.

Une autre technique consiste à provoquer une sclérose de la veine par introduction d'un produit sous forme de mousse. Cette technique a un coût de mise en oeuvre réduit et permet de traiter les veines collatérales. Elle a toutefois pour inconvénient de ne pas permettre de contrôler la répartition du produit sclérosant, ce de quoi il résulte des risques plus importants de phlébite profonde et d'embolie. De plus, cette technique est moins efficace en termes d'obturation de la veine, étant donné qu'elle n'agit pas au niveau du collagène pariétal comme les techniques précédentes mais uniquement sur la zone endo-veineuse. En outre, la fiabilité de l'occlusion obtenue est incertaine dans le temps, des reperméations de la veine traitée étant relativement fréquentes à moyen terme.

WO94/15534 décrit un dispositif d'occlusion d'un conduit corporel sous la forme d'un élément tubulaire comprenant des fibres, une portion de ces fibres étant radiopaque.

WO94/09705 décrit un dispositif d'occlusion d'un conduit corporel sous la forme d'un élément tubulaire comprenant des fibres, ledit élément tubulaire enfermant une bobine hélicoïdale en matériau radiopaque.

La présente invention vise à remédier à l'ensemble des inconvénients des techniques existantes.

Son objectif principal est donc de fournir un dispositif d'occlusion implantable selon une procédure relativement simple, rapide et peu onéreuse à mettre en oeuvre, pouvant être mise en oeuvre sous anesthésie locale, et n'induisant que peu de douleurs post-opératoires et pas de complications au niveau des tissus, en particulier au niveau des nerfs péri-saphéniens.

Un autre objectif de l'invention est de fournir un dispositif d'occlusion présentant des possibilités d'utilisation élargies, c'est-à-dire pouvant notamment être utilisé pour l'occlusion de la veine saphène interne mais aussi de la veine saphène externe et des veines collatérales de gros calibre.

Ces objectifs sont atteints par un dispositif d'occlusion formé par plusieurs fils tricotés ensemble de manière à constituer un tricot allongé "cylindrique", c'est-à-dire un tricot dans lequel les fils formant les mailles se croisent sensiblement au niveau de la zone radialement interne de ce tricot.

Le dispositif obtenu selon le procédé de fabrication de la présente invention a ainsi une structure à la fois maillée, faisant qu'il est compressible radialement et très souple longitudinalement, et "cylindrique", faisant que les fils sont présents au niveau de la zone radialement interne du dispositif. La compressibilité radiale et la souplesse du dispositif permettent l'introduction facile de ce dispositif dans le conduit à traiter, sous une simple anesthésie locale, et ne génèrent aucune rigidité sensible sous la peau ; la présence des fils au niveau de la zone radialement interne du dispositif, sur l'ensemble de la longueur du dispositif, permet d'assurer une occlusion fiable du conduit, sur une grande longueur de celui-ci. Dans le cas d'une veine variqueuse, le dispositif d'occlusion ne permet pas le passage direct du sang sur une longueur supérieure à une ou à quelques mailles ; il en résulte un ralentissement du flux sanguin conduisant à une thrombose de la veine, qui s'organise progressivement en fibrose.

Le dispositif obtenu d'occlusion selon le procédé de fabrication de la présente invention n'implique ainsi aucun échauffement du conduit corporel traité et n'induit aucun risque de lésion des nerfs sensitifs qui accompagnent les veines. Il peut dès lors être implanté selon une procédure relativement simple, rapide et peu onéreuse à mettre en oeuvre, et peut être utilisé pour les indications les plus larges, en particulier pour traiter les veines Saphènes externes, les récidives et les veines collatérales.

De préférence, le dispositif est constitué de fils en matériau résorbable, notamment en acide polylactique, polyglycolique ou un copolymère de ces derniers.

Grâce à la structure maillée précitée, le dispositif d'occlusion a un rapport poids/volume très faible (de l'ordre de 0,02 à 0,08 grammes par cm³ selon les différentes tailles de dispositifs), qui lui permet d'occuper l'ensemble du volume du conduit avec une faible quantité de fil par unité de longueur. Cette faible quantité de fil rend possible une résorption complète du dispositif d'occlusion, sans réaction inflammatoire cliniquement significative. Dans le cas du traitement d'une veine variqueuse, une fois la fibrose de la veine réalisée, les macrophages résorbent les résidus hydrolysés du dispositif d'occlusion et les thrombis fibreux ; la veine se réduit finalement à un cordon fibreux qui sera éliminé par l'organisme, tout comme est éliminée l'acide lactique et/ou glycolique.

Avantageusement, le matériau résorbable constituant les fils du dispositif d'occlusion, le nombre de ces fils et le diamètre de ces fils sont déterminés de telle sorte que la durée nécessaire à la résorption du dispositif d'occlusion soit supérieure ou égale à la durée de résorption naturelle du conduit corporel traité une fois l'occlusion réalisée.

Le dispositif d'occlusion peut être réalisé en mailles jetées ou cueillies, et est de préférence réalisé en mailles jetées.

Avantageusement, les fils formant le dispositif d'occlusion sont enduits d'une ou plusieurs substances de nature à favoriser l'occlusion et/ou la dégénérescence du conduit corporel traité, ou reçoivent un traitement de surface augmentant leur thrombogénéité. Il peut notamment s'agir d'un produit propre à irriter la paroi du conduit pour induire un spasme de celui-ci autour du dispositif. Dans le cas du traitement d'une veine variqueuse, ce spasme favorise l'occlusion de la veine et l'apparition rapide de la thrombose. Il peut s'agir également d'activateurs de la coagulation.

Le dispositif d'occlusion peut en outre comprendre au moins un fil longitudinal relié à ses extrémités, formant un moyen anti-allongement de ce dispositif lors de son introduction dans le tube longitudinal du dispositif de mise en place et lors de sa mise en place dans le conduit corporel.

Le dispositif d'occlusion permet ainsi de déposer dans le conduit corporel un dispositif d'occlusion ayant, à l'état non comprimé, un diamètre équivalent ou légèrement supérieur à celui de ce conduit corporel.

II est ainsi obtenu une densité suffisante de fil dans le conduit corporel, qui assure l'occlusion du conduit dans les meilleures conditions, et un degré d'agression de la paroi du conduit qui contribue efficacement à cette occlusion.

Le diamètre du dispositif d'occlusion peut notamment aller de 4 à 9 mm selon les veines à traiter, et le diamètre externe du tube peut aller de 3 à 4,5 mm.

Des moyens de préhension peuvent être constitués à partir des fils formant le dispositif d'occlusion, notamment par nouage ou autre regroupement, en particulier fusion, de ces fils au niveau de l'extrémité distale du,dispositif d'occlusion.

Pour sa bonne compréhension, l'invention est à nouveau décrite ci-dessous en référence au dessin schématique annexé représentant, à titre d'exemples non limitatifs, deux formes de réalisation possibles du dispositif d'occlusion.
La figure 1 est une vue de côté d'une portion du dispositif d'occlusion selon une première forme de réalisation, ce dispositif, tel que représenté, étant grossi environ quatre fois ;
la figure 2 est une vue très schématique des aiguilles d'un métier à tricoter à partir duquel ce dispositif est obtenu, et du parcours des fils alimentant ces aiguilles ;
la figure 3 est une vue de la grille de liage correspondante;
la figure 4 est une vue en coupe longitudinale dudit dispositif de mise en place qui ne fait pas partie de l'invention, ledit dispositif d'occlusion étant engagé à l'intérieur de ce dispositif de mise en place;
la figure 5 est une vue à échelle agrandie d'une incision pratiquée dans une jambe, permettant d'exposer la partie proximale de la veine variqueuse à traiter ;
la figure 6 est une vue de la jambe après engagement dans la veine à traiter du dispositif d'occlusion et du dispositif de mise en place;
la figure 7 est une vue similaire à la figure 6, après retrait du dispositif de mise en place;
la figure 8 est une vue du dispositif d'occlusion et du dispositif de mise en place selon la deuxième forme de réalisation, en coupe longitudinale ; et
la figure 9 est une vue du dispositif d'occlusion et du dispositif de mise en place similaire à la figure 8, le dispositif de mise en place présentant à son extrémité proximale un embout de connexion différent de celui du dispositif montré sur la figure 8.

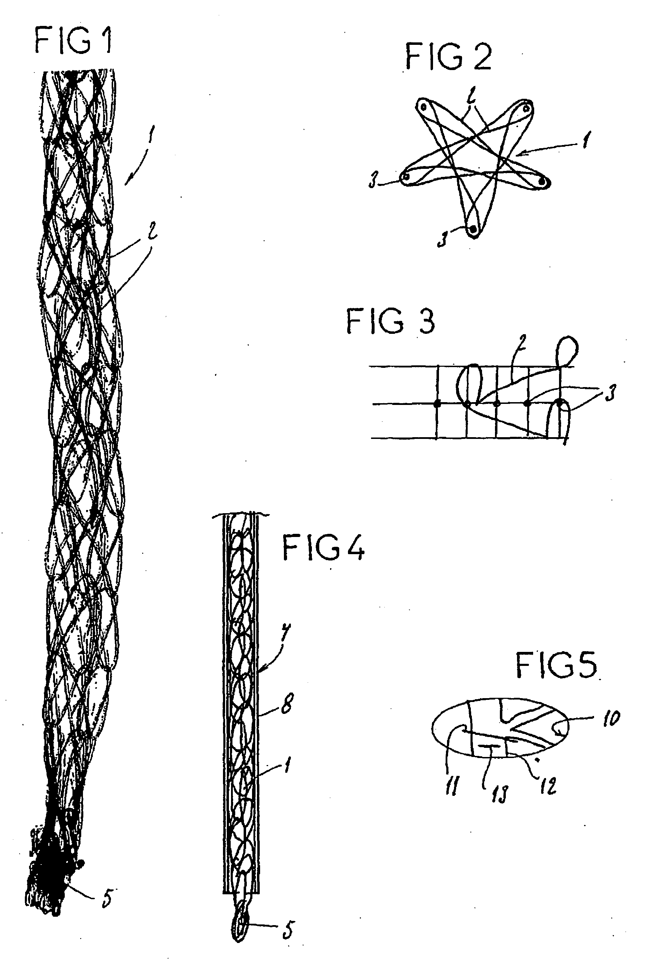

La figure 1 représente un dispositif d'occlusion 1 permettant le traitement d'une veine variqueuse.

On sait qu'une veine variqueuse est une veine qui n'est plus à même d'empêcher le reflux du sang en direction de l'extrémité du membre, c'est-à-dire en direction du pied dans le cas d'une jambe. Il en résulte un engorgement des tissus, et, lorsque la veine est très dilatée, le développement de thromboses.

La veine variqueuse doit alors être traitée de manière à empêcher la circulation du sang au travers d'elle ; les veines formant un réseau, le sang empruntera alors d'autres veines pour sa circulation.

Le dispositif 1 est formé par cinq monofils 2 en acide polylactique et/ou polyglycolique tricotés ensemble de manière à constituer un tricot allongé "cylindrique", c'est-à-dire un tricot dans lequel les fils 2 se croisent sensiblement au niveau de la zone radialement interne de ce tricot.

En référence aux figures 2 et 3, il apparaît que ce tricotage est réalisé sur un métier circulaire, qui est un métier chaîne. Ce tricotage est à mailles jetées. Le liage est obtenu en exécutant une grande amplitude avec les fils 2, permettant à ces derniers de sauter plusieurs aiguilles 3 ; un fil 2 alimente ainsi une aiguille 3 puis saute plusieurs aiguilles de manière à alimenter une seconde aiguille 3 distante angulairement de la première aiguille, de telle sorte que la portion de fil 2 située entre ces aiguilles s'étend au niveau de la zone radialement interne 4 de la surface circulaire délimitée par les aiguilles 3, qui est également la zone radialement interne du dispositif 1. Dans l'exemple représenté, le métier comprend cinq aiguilles 3 qui sont alimentées chacune par un fil 2. Par le mouvement du porte-fil, un fil 2 alimente une aiguille en maille ouverte puis saute deux aiguilles, de sorte qu'il alimente l'aiguille située en quatrième position par rapport à cette première aiguille, puis revient à la première aiguille, et le cycle recommence.

Comme cela apparaît sur la figure 1, le tricotin ainsi obtenu a une structure à la fois maillée, faisant qu'il est compressible radialement et très souple longitudinalement, et est "cylindrique", c'est-à-dire non tubulaire, faisant que les fils 2 sont présents au niveau de la zone radialement interne du dispositif 1, élément essentiel pour le caractère occlusif du dispositif 1.

A une extrémité du dispositif 1, les fils 2 sont réunis par exemple par thermofusion de manière à constituer une zone de préhension 5 de forme plus ou moins arrondie. Cette zone 5 est dimensionnée de manière à pouvoir être saisie au travers de la peau, ainsi que cela apparaîtra plus loin.

Le dispositif 1 est produit en une longueur suffisante pour permettent de traiter toute longueur de veine susceptible d'être affectée, par exemple 60 cm, et peut être coupé à la longueur désirée. Une thermofixation peut être réalisée pour limiter la déformabilité des mailles et éviter tout démaillage.

De plus, afin de faciliter ultérieurement l'introduction du dispositif d'occlusion 1 dans le dispositif 7 de mise en place, un ou plusieurs fils peuvent être "descendus" verticalement au centre du dispositif 1 pendant la phase de tricotage. Ces fils non maillés servent à limiter l'effet de la traction lors de l'introduction du dispositif d'occlusion 1 dans le dispositif de mise en place 7, et évitant ainsi une trop grande déformation des mailles. Ce ou ces fils longitudinaux évitent aussi l'allongement du dispositif 1 lors de son larguage dans la veine.

En référence à la figure 4, il apparaît que le dispositif 7 de mise en place du dispositif 1 comprend un tube 8, propre à contenir ce dispositif 1, avec toutefois la zone 5 dépassant d'une extrémité de ce tube 8. Ce tube 8 est en polyéthylène et a une épaisseur telle qu'il est suffisamment rigide pour permettre son engagement par coulissement dans la veine à traiter mais suffisamment souple pour ne pas être blessant et pour permettre une progression aisée dans les vaisseaux sinueux.

En pratique, ainsi que cela est illustré par les figures 5 à 7, une incision 10 est pratiquée dans la jambe de manière à exposer la jonction Saphèno-fémorale au Scarpa, ou à exposer la crosse Saphène externe derrière le genou ; une ligature 11 non résorbable est mise en place soit au niveau de la jonction Saphèno-fémorale si un reflux sanguin a été constaté à ce niveau, soit en dessous de cette jonction si l'on souhaite préserver le drainage des veine sous-cutanées abdominales et honteuses ; une incision 13 est ensuite réalisée dans la veine 12 à traiter, en dessous de la ligature 11, et le dispositif 7, dans lequel est introduit le dispositif 1 de la manière précitée, est engagé dans la veine 12 sur la longueur nécessaire ; une fois cet engagement réalisé, la zone 5 est saisie à travers la peau puis le tube 8 est retiré de manière à libérer le dispositif 1 dans la veine 12 ; l'incision 13 puis l'incision 10 sont refermées.

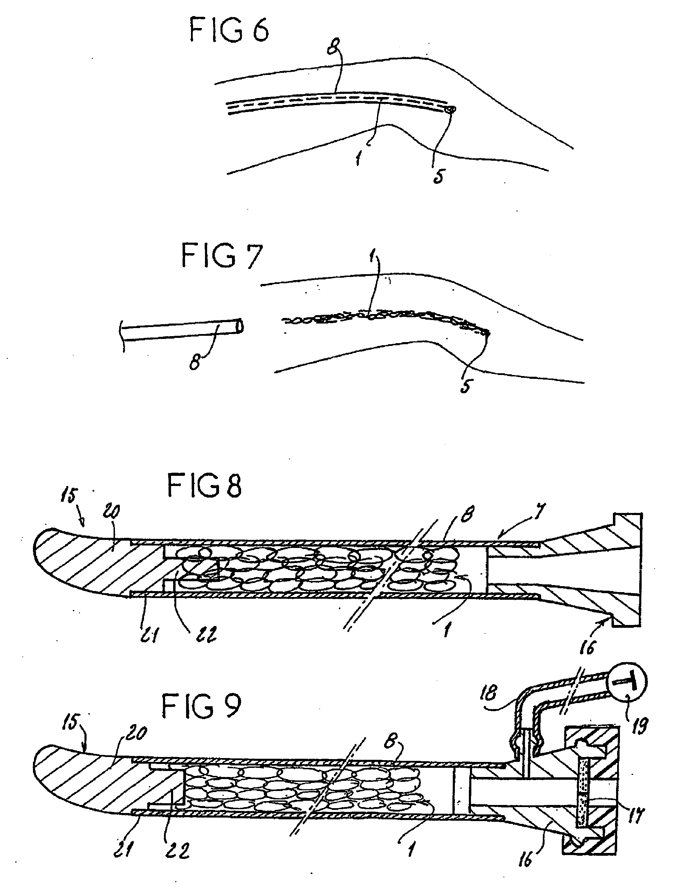

La figure 8 montre schématiquement un dispositif 1 identique à celui qui vient d'être décrit, sinon qu'il ne présente pas la zone 5, et un dispositif 7 comprenant, outre le tube 8, un embout distal 15 et un embout proximal 16. Ce dispositif ne constitue pas non plus l'objet revendiqué.

L'embout 15 présente une portion arrondie 20, conformée pour être atraumatique, une portion circulaire 21 de diamètre légèrement inférieur au diamètre interne du tube 8, et une patte axiale 22 percée d'un trou. La portion 21 peut être engagée dans le tube 8 et permet le montage de l'embout 15 sur l'extrémité distale du tube 8, tandis que la patte 22 permet de lier cet embout 15 au dispositif 1.

Cet embout 15 remplit la même fonction que la zone 5 précitée. Après retrait du tube 8, il est extrait du corps du patient par une incision distale et une séparation d'avec le dispositif 1. Il peut également être réalisé en un matériau résorbable à résorption rapide et être alors laissé en place.

L'embout 16 est, dans l'exemple représenté sur la figure 8, de type "Luer lock". Il peut également être de type raccord en "Y" avec des extrémités au standard "Luer lock". Cet embout 16 permet une injection de fluide à l'intérieur du tube 8, soit préalablement à la mise en place du dispositif 1, soit pendant cette mise en place.

La figure 9 montre un dispositif 7 identique à celui de la figure 8 mais avec, à son extrémité proximale, un embout 16 de type embase avec valve hémostatique 17 et une voie latérale 18 terminée en son extrémité par un robinet 19 à deux ou trois voies. L'injection de fluide se fait par cette voie latérale.

Ainsi qu'il apparaît de ce qui précède, l'invention apporte une amélioration déterminante à la technique antérieure, en fournissant un dispositif d'occlusion 1 d'un conduit corporel, notamment d'une veine variqueuse 12, qui est implantable selon une procédure relativement simple, rapide et peu onéreuse à mettre en oeuvre, cette procédure pouvant être mise en oeuvre sous anesthésie locale et n'induisant que peu de douleurs post-opératoires et pas de complications au niveau des tissus. Ce dispositif d'occlusion présente en outre des possibilités d'utilisation élargies, c'est-à-dire qu'il peut notamment être utilisé pour l'occlusion de la veine Saphène interne mais aussi de la veine Saphène externe et des veines collatérales de gros calibre.

Il va de soi que l'invention n'est pas limitée à la forme de réalisation décrite ci-dessus à titre d'exemple mais qu'elle en embrasse au contraire toutes les variantes de réalisation entrant dans le champ de protection défini par les revendications ci-annexées.

## Revendications

1. - Procédé de fabrication d'un dispositif d'occlusion d'un conduit corporel, notamment d'une veine variqueuse, **caractérisé en ce qu'**il comprend l'étape de tricoter plusieurs fils (2) ensemble de manière à constituer un tricot allongé cylindrique, c'est-à-dire un tricot dans lequel les fils (2) formant les mailles se croisent sensiblement au niveau de la zone radialement interne de ce tricot, ledit tricot étant réalisé sur un métier circulaire à aiguilles, les fils sautant plusieurs aiguilles de telle sorte que les portions des fils situées entre ces aiguilles s'étendent au niveau de la zone radialement interne de la surface circulaire délimitée par les aiguilles, qui est également la zone radialement interne du dispositif.

2. - Procédé selon la revendication 1, **caractérisé en ce que** le dispositif d'occlusion est constitué de fils (2) en matériau résorbable, notamment en acide polylactique, polyglycolique ou un copolymère de ces derniers.

3. - Procédé selon la revendication 2, **caractérisé en ce que** le matériau résorbable constituant les fils (2), le nombre de ces fils (2) et le diamètre de ces fils (2) sont déterminés de telle sorte que la durée nécessaire à la résorption du dispositif d'occlusion soit supérieure ou égale à la durée de résorption naturelle du conduit corporel traité une fois l'occlusion réalisée.

4. - Procédé selon l'une des revendications 1 à 3, **caractérisé en ce que** le dispositif d'occlusion est réalisé en mailles jetées.

5. - Procédé selon l'une des revendications 1 à 4, **caractérisé en ce que** les fils (2) qui forment le dispositif d'occlusion sont enduits d'une ou plusieurs substances de nature à favoriser l'occlusion et/ou la dégénérescence du conduit corporel traité, ou reçoivent un traitement de surface augmentant leur thrombogénéité.

6. - Procédé selon l'une des revendications 1 à 5, **caractérisé en ce qu'**une thermofixation est réalisée pour limiter la déformabilité des mailles et éviter tout démaillage.

7. - Procédé selon l'une des revendications 1 à 6, **caractérisé en ce qu'**au moins un fil longitudinal est descendu verticalement au centre du dispositif pendant la phase de tricotage.

8. - Procédé selon la revendication précédente, **caractérisé en ce que** le fil longitudinal est relié aux extrémités du dispositif d'occlusion, formant un moyen anti-allongement du dispositif d'occlusion (1).

9. - Procédé selon l'une quelconque des revendications précédentes, **caractérisé en ce qu'**à une extrémité du dispositif (1), les fils (2) sont réunis par thermofusion de manière à constituer une zone de préhension (5) de forme arrondie.

## Claims

1. Process for the manufacture of a device for occlusion of a corporeal duct, in particular a varicose vein, **characterized in that** it comprises the step of knitting several yarns (2) together so as to constitute a cylindrical elongate knitted fabric, that is to say a knitted fabric in which the yarns (2) forming the stitches intersect substantially at the radially inner zone of this knitted fabric, said knitted fabric being done on a needle circular knitting machine, the yarns jumping several needles so that the portions of the yarns situated between these needles extend at the radially inner zone of the circular surface delimited by the needles, which is also the radially inner zone of the device.

2. Process as claimed in claim 1, **characterized in that** the occlusion device is made up of yarns (2) of absorbable material, in particular of polylactic acid, polyglycolic acid, or a copolymer of these.

3. Process as claimed in claim 2, **characterized in that** the absorbable material constituting the yarns (2), the number of these yarns (2) and the diameter of these yarns (2) are determined in such a way that the period needed for absorption of the occlusion device is greater than or equal to the period of natural absorption of the treated corporeal duct once the occlusion has been performed.

4. Process as claimed in one of claims 1 through 3, **characterized in that** the occlusion device is made of warp stitches.

5. Process as claimed in one of claims 1 through 4, **characterized in that** the yarns (2) forming it are coated with one or more substances of a nature to promote occlusion and/or degeneration of the treated corporeal duct, or their surface is treated to increase their thrombogenicity.

6. Process as claimed in one of claims 1 through 5, **characterized in that** thermosetting is performed in order to limit the deformability of the stitches and to prevent unraveling.

7. Process as claimed in one of claims 1 through 6, **characterized in that** at least one longitudinal yarn can be descended vertically at the center of the device during the knitting phase.

8. Process as claimed in the preceding claim, **characterized in that** the longitudinal yarn is connected at the ends of the occlusion device, forming a means acting against lengthening of the occlusion device (1).

9. Process as claimed in any one of the preceding claims, **characterized in that** at one end of the device (1), the yarns (2) are joined together by thermal bonding so as to constitute a grip zone (5) of more or less rounded shape.

## Patentansprüche

1. Verfahren zum Herstellen einer Okklusionsvorrichtung für ein Körpergefäß, insbesondere eine Krampfader, **dadurch gekennzeichnet, dass** es den Schritt des Zusammenstrickens mehrerer Fäden (2) umfasst, um ein zylindrisches längliches Gestrick zu bilden, d.h. ein Gestrick, bei dem die Fäden (2), welche die Maschen bilden, sich im Wesentlichen an der radial inneren Zone dieses Gestricks kreuzen, wobei das Gestrick auf einer Rundwirkmaschine mit Nadeln angefertigt wird, wobei die Fäden derart mehrere Nadeln überspringen, dass sich die Abschnitte der Fäden, die sich zwischen diesen Nadeln befinden, an der radial inneren Zone der von den Nadeln begrenzten kreisförmigen Oberfläche erstrecken, die auch die radial innere Zone der Vorrichtung ist.

2. Verfahren nach Anspruch 1, **dadurch gekennzeichnet, dass** die Okklusionsvorrichtung aus Fäden (2) aus resorbierbarem Material, insbesondere aus Polymilchsäure, Polyglykolsäure oder einem Copolymer derselben, besteht.

3. Verfahren nach Anspruch 2, **dadurch gekennzeichnet, dass**, wenn das resorbierbare Material die Fäden (2) bildet, die Anzahl dieser Fäden (2) und der Durchmesser dieser Fäden (2) derart bestimmt sind, dass die Dauer, die zur Resorption der Okklusionsvorrichtung notwendig ist, größer oder gleich der natürlichen Resorptionsdauer des behandelten Körpergefäßes ist, nachdem die Okklusion ausgebildet wurde.

4. Verfahren nach einem der Ansprüche 1 bis 3, **dadurch gekennzeichnet, dass** die Okklusionsvorrichtung aus Kettenmaschen angefertigt wird.

5. Verfahren nach einem der Ansprüche 1 bis 4, **dadurch gekennzeichnet, dass** die Fäden (2), welche die Okklusionsvorrichtung bilden, mit einer oder mehreren Substanzen beschichtet sind, die derart beschaffen sind, dass sie die Okklusion und/oder die Degeneration des behandelten Körpergefäßes begünstigen, oder eine Oberflächenbehandlung erhalten, die ihre Thrombogenität erhöht.

6. Verfahren nach einem der Ansprüche 1 bis 5, **dadurch gekennzeichnet, dass** eine Thermofixierung ausgeführt wird, um die Verformbarkeit der Maschen einzuschränken und ein Aufziehen zu vermeiden.

7. Verfahren nach einem der Ansprüche 1 bis 6, **dadurch gekennzeichnet, dass** während der Strickphase mindestens ein Längsfaden senkrecht bis zur Mitte der Vorrichtung eingeführt wird.

8. Verfahren nach dem vorhergehenden Anspruch, **dadurch gekennzeichnet, dass der Längsfaden mit den Enden der** Okklusionsvorrichtung verbunden ist, wodurch er ein Mittel gegen eine Streckung der Okklusionsvorrichtung (1) bildet.

9. Verfahren nach einem der vorhergehenden Ansprüche, **dadurch gekennzeichnet, dass** die Fäden (2) an einem Ende der Vorrichtung (1) durch Heißverschmelzen zusammengeführt sind, um eine abgerundete Griffzone (5) zu bilden.
